# EUROPEAN PATENT APPLICATION

(11) **EP 4 261 525 A1**
(43) Date of publication of application: **18.10.2023**
(21) Application number: 22460071.8
(22) Date of filing: 15.12.2022
(51) Int. Cl.: G01N 17/02, G01N 33/38, G01N 33/00

(54) **HYBRID SENSOR AND METHOD FOR MEASURING CORROSION RATE OF REINFORCEMENT, CONDUCTIVITY, AND TEMPERATURE OF CONCRETE**

(30) Priority: 23.02.2022 PL 44044622
(71) Applicant: Politechnika Slaska, 44-100 Gliwice (PL)
(72) Inventor: Jasniok, Tomasz, 43-190 Mikolów (PL)

(57) **Abstract**

A hybrid sensor for measuring corrosion rate of reinforcement (10), conductivity, and temperature of concrete is equipped with two identical modules (6) positioned opposite one another, wherein each module (6) contains working electrodes (1) and auxiliary electrodes (2), which are embedded alternately one above the other in the concrete, a fixed reference electrode (4) on a side of the module (6) with the working and the auxiliary electrodes (1, 2), and temperature sensors (5) with an operating range from -30°C to 100°C located adjacent to the working electrodes (1) within a distance not greater than 10 mm. A method for measuring corrosion rate of reinforcement (10), conductivity, and temperature of concrete using the hybrid sensor for measuring corrosion rate of reinforcement (10), conductivity, and temperature of concrete is also disclosed.

## Description

The present invention relates to a hybrid sensor and a method for measuring corrosion rate of reinforcement, concrete conductivity and temperature for monitoring corrosion in reinforced concrete civil engineering structures.

Concrete structures are exposed to various external factors, of which carbon dioxide and chlorides specified in *PN-EN 206*+*A2:2021-08 Concrete* - *Requirements, properties, production and compliance* present the major corrosion hazard to reinforcing steel in concrete. When reinforcement corrosion is initiated, its intensity is affected by physical factors, such as concrete temperature and conductivity as described in the paper *Ściślewski Z.: Ochrona konstrukcji zelbetowych. Wydawnictwo "Arkady", Warszawa, 1999.* Reinforcement corrosion in concrete reduces load capacity of a reinforced concrete member, which results in failure or even the structural collapse, therefore it is essential to properly identify corrosion risk for reinforcement and precisely measure factors that affect its intensity.

According to *Zybura A., JasniokM., Jaśniok T.: Diagnostyka konstrukcji zelbetowych. T. 2. Badania korozji zbrojenia i w* 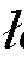 *aściwości ochronnych betonu, Wydawnictwo Naukowe PWN, Warszawa, 2011* for more than 30 years the methods employed in electrochemical laboratories, where the best results were obtained with the Linear Polarization Resistance (LPR), Electrochemical Impedance Spectroscopy (EIS) or Galvanostatic Pulse (GP) method, have been adopted to reinforced concrete structures to determine corrosion rate of reinforcement in concrete. The above methods are used to obtain corrosion current density, which according to Faraday's law, determines mass decrement of rebars, and thus is used to determine a decreasing capacity loss of reinforced concrete elements over time. Due to high variability of the corrosion rate of reinforcement, it can change more than several dozen times over few weeks as presented in the paper by Andrade C., Alonso C.: Corrosion rate monitoring in the laboratory and on-site. Construction and Building Materials. 1996 . vol. 10. pp. 315-328*,* therefore tests conducted on the site at specific ambient temperature, relative humidity, the building age, and concentration of aggressive substances inducing corrosion do not provide representative results because it cannot be determined whether the obtained data are close to maximum, minimum or average values. Only tests on corrosion rate over time in variable environmental conditions provide records of amplitude of those changes and may be used to determine dominant values. Distribution of tests over time is connected with monitoring corrosion of the structure.

Continuous monitoring of corrosion rate determined from electrochemical measurements, concrete conductivity and its temperature provide the complete picture of corrosion level and variability of corrosion rate, and factors which affect these values.

Systems of continuous monitoring with sensors to measure densities of corrosion current, conductivity and temperature of concrete have been already used in concrete structures as described in the paper by Duffó G.S., Farina S.B. Development of an embeddable sensor to monitor the corrosion process of new and existing reinforced concrete structures, Construction and Building Materials. 2009 . vol. 23. pp. 2746-2751 and Andrade C., Martinez I. Corrosion Rate Monitoring of Deteriorated and Repaired Structures Through on-Site Linear Polarization Measurements Using Surface or Embedded Sensors. 2nd International Symposium on Advances in Concrete through Science and Engineering 11-13 September 2006, Quebec City, Canada*.*

Corrosion sensors and methods for measuring corrosion in a reinforced concrete structure that involve inserting the corrosion sensor into the reinforced concrete structure as in the above solutions are known from patent descriptions US2011259092A1 and CN207557160U*,* but the sensors are additionally equipped with sensors for measuring concentrations of chloride ions and pH values. The described solutions are presented in the papers by Yu H., Caseres L.: An embedded multi-parameter corrosion sensor for reinforced concrete structures. Materials and Corrosion. 2012 . vol. 63. pp. 1011-1016 oraz Dong S.G. et al. Effective monitoring of corrosion in reinforcing steel in concrete constructions by a multifunctional sensor, Electrochimica Acta. 2011. vol. 56. pp. 1881-1888*.*
Monitoring the rate and intensity of corrosion with sensors at a certain depth of concrete cover, where a working electrode of the sensor employed for electrochemical measurements is placed, is a disadvantage of the above solutions.
Therefore, it is not possible to obtain information on a progressive loss of protective properties of concrete against reinforcement,

The method of early informing about developing contamination of concrete by means of a sensor for monitoring corrosions of steel bars in a reinforced concrete member composed of a series of electrodes at different lengths of concrete cover and determining the level of concrete contamination based on changes in current intensity in a cell in particular electrodes is known from patent description CN100454000C*.* The electrodes are fixed to a stepped base of the sensor. The monitoring sensor for collecting data on a regular basis is embedded in concrete during the construction. When a corroded steel bar in a certain electrode rod is detected, the steel bar is protected against corrosion with a cathodic protection device.

The solution, in which corrosive environment mainly of ground is detected by employing sensors in the series configuration, consisting of a working electrode made of corrodible metal material in the tested environment and a non-corrodible counter electrode made of noble metal, is known from patent description JP2013160541A. Short-circuiting of both metals by moisture content in the ground creates cells which cause current flow, whose intensity is measured by each sensor with a sensitive ammeter - the highest the current is, the more corrosive the ground is.

The technical problem to be solved is development of a new corrosion sensor for continuous monitoring of reinforced concrete structures, consisting of many steel electrodes placed at different depths of concrete cover, which measure the rate to determine satisfactory or poor protective properties of concrete against steel, which initiate corrosion processes . Such tests eliminate the need for determining the current concentration of chlorides and concrete pH because they comprehensively include all factors inducing corrosion. In addition, the corrosion sensor should provide measurements at different levels of current conductivity and temperature of concrete. Conductivity and temperature correlated with changes of corrosion rate may be used to predict period, after which concrete losses its protective properties against steel at the depth of existing rebars.

The hybrid sensor for corrosion rate of reinforcement, conductivity and temperature of concrete is characterized in that it is equipped with two identical modules positioned opposite one another, wherein each module contains working and auxiliary electrodes embedded alternately one above the other, the fixed reference electrode on a side of the column with the working and the auxiliary electrodes, and temperature sensors with an operating range from -30°C to 100°C placed at a height of the working electrodes within a distance not greater than 10 mm.

Preferably, in the hybrid sensor according to the present invention, the distance between the modules is from 20 mm to 100 mm.

Preferably, in the hybrid sensor according to the present invention, the working electrode is made of quality, reinforcing carbon steel with a thickness not greater than 5 mm, preferably 2 mm.

Preferably, in the hybrid sensor according to the present invention, the auxiliary electrode is made of metal resistant to electrochemical corrosion, with a thickness not greater than 5 mm, preferably 2 mm.

Preferably, in the hybrid sensor according to the invention, the reference electrode is made of graphite, and its height is equal to a height of the column of alternately placed working electrodes and auxiliary electrodes.

Preferably, in the hybrid sensor according to the present invention, a thermocouple or resistance temperature detector is used as the temperature sensor.

Preferably, in the hybrid sensor according to the present invention the working, auxiliary, and reference electrodes have one common, flat surface, and other surfaces are electrically insulated from one another and from concrete with resin, preferably the epoxy resin.

Preferably, in the hybrid sensor according to the present invention, the distance between the working electrode and the auxiliary electrode is not greater than 10 mm, preferably 1 mm.

Preferably, in the hybrid sensor according to the present invention, the distance between the reference electrode and the working electrode and the auxiliary electrode is not greater than 10 mm, preferably 2 mm.

Preferably, in the hybrid sensor according to the present invention, the distance between the moduli surfaces and concrete is not greater than 10 mm, preferably 3÷5 mm.

The method for measuring corrosion rate of reinforcement, conductivity and temperature of concrete consists in the fact that the working electrodes, and the associated temperature sensors and the auxiliary electrodes are placed alternately in a form with spacers, to which the reference electrode is fixed, wherein wires are connected to all the electrodes and temperature sensors, followed by insulating all the electrodes and sensors with resin and forming a rectangular module, one wall of which is ground down and polished until whole bases of the working electrodes, the auxiliary electrodes and a wall of the reference electrode are uncovered, which is fixed to the base within a distance of 40 mm from the same module, the whole reinforcement system for a reinforced concrete member is stabilized with steel clamping plates, followed by connecting the working electrode, the auxiliary electrode, and the reference electrode to the potentiostat and taking measurement with the electrochemical polarization method, and connecting the auxiliary electrodes to a conductometer to measure concrete conductivity.

Preferably, in the method for measuring corrosion rate according to the present invention, the method of: linear polarization resistance (LPR), electrochemical impedance spectroscopy (EIS), or galvanostatic pulse (GP) is used as the electrochemical polarization method.

The advantage of this solution according to the present invention is continuous monitoring of corrosion rate of steel in the sensor using the electrochemical methods at any depth of concrete cover thickness while simultaneously measuring concrete conductivity and temperature at these depths. The above information obtained in set time intervals is the basis for predicting the beginning and degree of corrosion of the existing reinforcement in the monitored structure, which allows for undertaking relevant measures to prevent deterioration of technical conditions of the structure.

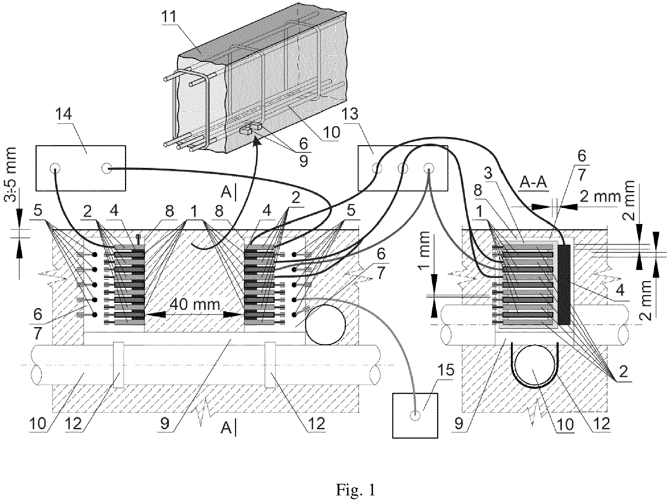

The object of the invention in the embodiment is illustrated in the drawing as a diagram of the hybrid sensor for corrosion rate of reinforcement, conductivity and temperature of concrete.
The hybrid sensor for corrosion rate of reinforcement, conductivity and temperature of concrete is equipped with two identical modules **6** positioned opposite one another, within a distance from 20 mm to 100 mm, wherein each module **6** consists the working electrodes **1** and the auxiliary electrodes **2**, which are embedded alternately one above another, the fixed reference electrode **4** on a side of the column with the electrodes **1** and **2**, and the temperature sensors **5** within an operating range from - 30°C to 100°C placed at the height of the working electrodes **1** within a distance not greater than 10 mm. The working electrodes **1** are made of quality, reinforcing carbon steel with a thickness not greater than 5 mm, preferably 2 mm. The auxiliary electrodes **2** are made of metal resistant to electrochemical corrosion, with a thickness not greater than 5 mm, preferably 2 mm. The reference electrode **4** is made of graphite, and its height is equal to a height of alternately positioned electrodes **1** and **2.** The electrodes **1**, **2**, and **4** have one common, flat surface, and their other surfaces are electrically insulated from one another and from concrete with resin 7, preferably epoxy resin. The distance between the electrodes **1** and **2** is from 0.5 to 10 mm, preferably 1 mm. The distance between the reference electrode **4** and all other electrodes **1** and **2** is not greater than 10 mm, preferably 2 mm. The distance between the top surface of modules **6** and concrete surface is not greater than 10 mm, preferably 3÷5 mm. The thermocouple or resistance temperature detector is used as the temperature sensor **5**.
The working electrode **1** in the form of a strip of reinforcing steel grade B500SP with a thickness of 2 mm and a width of 20 mm and the auxiliary electrode **2** in the form of a strip of stainless steel grade
X5CrNi18-10 with a thickness of 2 mm are placed alternately in the **form 3** with spacers of 1 mm in thickness, wherein the auxiliary electrode **2** is the first and the last steel strip. The reference electrode **4** in the form of an oblong graphite cuboid with the square baseplate 4×4 mm and a height equal to the distance between the first and the last auxiliary electrode **2** is fixed to a side wall of the form **3** within a distance of 2 mm. The thermocouple used as the temperature sensor **5** is fixed to each working electrode **1**. Wires are connected to all the electrodes **1**, **2** and **4** and the temperature sensor **5**, and then they are stabilized and simultaneously electrically insulated from one another in one rectangular module **6** using epoxy resin **7**. One wall **8** of the module **6** with resin **7** is ground until whole bases of the working electrodes **1**, the auxiliary electrodes **2** and the wall of the reference electrode **4** are uncovered, further it is polished to achieve the roughness class Ra 0.63 according to standard PN-EN ISO 1302:2004. After preparing two modules **6** with polished walls **8**, they are fixed to a plastic base **9** to arrange the walls **8** opposite one another. Fixing of the modules **6** to the base **9** precisely determines the distance between them, which is equal to 40 mm. The base **9** and the modules **6** are fixed to the reinforcement **10** of the reinforced concrete member **11** and stabilized using the steel clamping plates **12**. The upper part of the modules **6** should be placed approx. 3=5 mm below concrete surface. When concrete is poured into formwork and totally dried, a selected working electrode **1** is connected with two adjacent auxiliary electrodes **2** and the reference electrode **4** to the potentiostat **13** in any of the modules **6** and measurement is taken with the method of
Linear Polarization Resistance (LPR), Electrochemical Impedance Spectroscopy (EIS), or Galvanostatic Pulse (GP). It is possible to connect any two auxiliary electrodes **2** to the conductometer **14** at the same depth in both modules **6** and measure concrete conductivity at the tested depth of concrete cover thickness. Additionally, by connecting a selected temperature sensor **5** to the gauge **15**, temperature may be measured at the selected depth.

## Claims

1. A hybrid sensor for corrosion rate of reinforcement, conductivity and temperature of concrete **characterized in that** it is equipped with two identical modules **6** placed opposite one another, wherein each module **6** contains working electrodes **1** and auxiliary electrodes **2**, which are embedded alternately one above the other, a fixed reference electrode **4** on a side of the column with the working and the auxiliary electrodes **1** and **2**, and temperature sensors **5** with an operating range from -30°C to 100°C located at a height of the working electrodes **1** within a distance not greater than 10 mm.

2. A hybrid sensor, according to claim 1, **characterized in that** the distance between the modules **6** is from 20 mm to 100 mm, preferably 40 mm.

3. A hybrid sensor, according to claim 1, **characterized in that** the working electrode **1** is made of quality, reinforcing carbon steel with a thickness not greater than 5 mm, preferably 2 mm.

4. A hybrid sensor according to claim 1, **characterized in that** the auxiliary electrode **2** is made of metal resistant to electrochemical corrosion, with a thickness not greater than 5 mm, preferably 2 mm.

5. A hybrid sensor according to claim 1, **characterized in that** the reference electrode **4** is made of graphite, and its height is equal to a height of the column of alternately placed working electrodes and auxiliary electrodes **1** and **2.**

6. A hybrid sensor according to claim 1, **characterized in that** the thermocouple or resistance temperature detector is used as the temperature sensor **5.**

7. A hybrid sensor according to claim 1, **characterized in that** the electrodes **1**, **2**, and **4** have one common, flat surface, and their other surfaces are electrically insulated from one another and from concrete with resin **7**, preferably epoxy resin.

8. A hybrid sensor, according to claim 1, **characterized in that** the distance between the electrodes **1**, **2** does not exceed 10 mm, preferably 1 mm.

9. A hybrid sensor, according to claim 1, **characterized in that** the distance between the reference electrode **4** and the working electrode **1** and the auxiliary electrode **2** is not greater than 10 mm, preferably 2 mm.

10. A hybrid sensor, according to claim 1, **characterized in that** the distance between the top surface of modules **6** and concrete surface is not greater than 10 mm, preferably 375 mm.

11. The method for measuring corrosion rate of reinforcement, conductivity and temperature of concrete **characterized in that** the working electrodes **1**, and the associated temperature sensors **5** and the auxiliary electrodes **2** are placed alternately in the form **3** with spacers, to which the reference electrode **4** is fixed, wherein wires are connected to all the electrodes and temperature sensors, followed by insulating all the electrodes and sensors with resin **7** and forming a rectangular module **6**, one wall **8** of which is ground down and polished until whole bases of the working electrodes **1**, the auxiliary electrodes **2** and a wall of the reference electrode **4** are uncovered, which is fixed to the base **9** within a distance of 40 mm from the same module **6**, the whole reinforcement system **10** for the reinforced concrete member is stabilized with the steel clamping plates **12**, followed by connecting the working electrode **1**, the auxiliary electrode **2**, and the reference electrode **4** to the potentiostat **13** and taking measurement with the electrochemical polarization method. and connecting the auxiliary electrodes **2** to the conductometer **14** to measure concrete conductivity.

12. A method for measuring corrosion rate according to claim 11, **characterized in that** the method of: linear polarization resistance (LPR), electrochemical impedance spectroscopy (EIS), or galvanostatic pulse (GP) is used as the electrochemical polarization method.
